(19) Europäisches Patentamt
European Patent Office
Office européen des brevets

(11) **EP 4 474 473 A1**

(12) **EUROPEAN PATENT APPLICATION**
published in accordance with Art. 153(4) EPC

(43) Date of publication:
**11.12.2024 Bulletin 2024/50**

(21) Application number: **23747007.5**

(22) Date of filing: **26.01.2023**

(51) International Patent Classification (IPC):
**C12N 5/07** (2010.01)   **C12N 5/071** (2010.01)
**C12M 1/00** (2006.01)   **C12M 1/42** (2006.01)

(52) Cooperative Patent Classification (CPC):
**C12M 1/00; C12M 1/42; C12N 5/06**

(86) International application number:
**PCT/JP2023/002363**

(87) International publication number:
**WO 2023/145797 (03.08.2023 Gazette 2023/31)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **31.01.2022   JP 2022013181
29.11.2022   JP 2022190039**

(71) Applicant: **CANON KABUSHIKI KAISHA**
**Tokyo 146-8501 (JP)**

(72) Inventors:
• **YAMAUCHI Fumio**
**Tokyo 146-8501 (JP)**

• **KANAZAKI Kengo**
**Tokyo 146-8501 (JP)**
• **MOCHIZUKI Shinsuke**
**Tokyo 146-8501 (JP)**
• **TSUBAKI Keiichiro**
**Tokyo 146-8501 (JP)**
• **KATO Mai**
**Tokyo 146-8501 (JP)**
• **FURUI Takaaki**
**Tokyo 146-8501 (JP)**
• **YOSHIDA Ryoichi**
**Tokyo 146-8501 (JP)**

(74) Representative: **Canon Europe Limited**
**European Intellectual Property Group**
**4 Roundwood Avenue**
**Stockley Park**
**Uxbridge UB11 1AF (GB)**

(54) **CELL DETACHMENT METHOD, CELL DETACHMENT SYSTEM, AND INFORMATION PROCESSING DEVICE**

(57)     A cell detachment method for detaching a cell from a substrate by applying vibration to the cell adherent to the substrate includes an incubation step of incubating the cell after bringing a cell detachment liquid containing substantially no proteolytic enzyme into contact with the cell, an information acquisition step of acquiring information relating to the incubated cell, a determination step of determining a time when vibration is applied to the incubated cell in accordance with the information relating to the cell, and a detachment step of applying vibration to the incubated cell.

FIG. 1

```
        ┌──────────┐
        │  START   │
        └──────────┘
             │
  ┌───────────────────────┐
  │    INCUBATING CELL    │──── S101
  └───────────────────────┘
             │
  ┌───────────────────────┐
  │  ACQUIRING INFORMATION│──── S102
  │    RELATING TO CELL   │
  └───────────────────────┘
             │
  ┌───────────────────────┐
  │ DETERMINING TIME WHEN │──── S103
  │ ULTRASONIC WAVE IS APPLIED │
  └───────────────────────┘
             │
  ┌───────────────────────┐
  │APPLYING ULTRASONIC WAVE TO CELL│──── S104
  └───────────────────────┘
             │
        ┌──────────┐
        │   END    │
        └──────────┘
```

EP 4 474 473 A1

## Description

Technical Field

The present invention relates to a cell detachment method, a cell detachment system, and an information processing device.

Background Art

In recent years, regarding development of drugs, a large paradigm shift from low molecular weight drugs and antibody drugs in the related art to cell medicine in which a cell itself is used as a drug and regenerative medicine which intends regeneration of tissues and organs has progressed. Regarding the cell medicine and the regenerative medicine, a large number of cells are necessary, and it is desired to efficiently stably supply an adhesive cell which occupies most of a biological tissue.

In culture of the adhesive cell, a target cell is acquired through each step of culturing a cell on a culture substrate such as a polystyrene dish, detaching the cell from the substrate, and recovering and washing the cell. When it is intended to further grow the cell, a so-called passage operation in which a portion of the acquired cell is transferred to a new substrate and cultured is performed. Regarding a detachment step of detaching the cell from the substrate in a series of the steps, investigation of efficient, stable supply of the cell has been performed.

In general, proteolytic enzymes such as trypsin are widely used for detaching a cell. In this method, a protein involving in a bond between the cell and the substrate is decomposed by action of the proteolytic enzyme, and thereafter the cell is completely detached by an operation such as tapping or pipetting. However, according to this method, many cell surface proteins are simultaneously decomposed by the proteolytic enzyme, and there is a concern that the quality of the cell may deteriorate. In addition, since the operation such as tapping or pipetting significantly changes in accordance with the proficiency level of a person, there is a problem relating to stable supply.

Citation List

Patent Literature

PTL 1: International Publication No. 2016/047368
PTL 2: Japanese Patent Laid-Open No. 2003-235540
PTL 3: International Publication No. 2007/136073

Summary of Invention

Technical Problem

Regarding the above-described problem, there is a known method in which a cell is detached from a culture container without using a proteolytic enzyme. PTL 1 proposes a method in which an ultrasonic wave is regioselectively incident on a cell to detach the cell from the container. However, a method for determining the timing of applying the ultrasonic wave is not disclosed.

Regarding cell culture, PTL 2 and PTL 3 disclose a method or a device for acquiring information of a cell area, brightness of a cell image, and the like and determining whether the cell is detached. However, a method for acquiring cell information of the cell in an adherent state and determining the timing of applying an ultrasonic wave is not disclosed.

Solution to Problem

In an adherent cell detachment method by using an ultrasonic wave in the related art, the cell remains adherent to a substrate, and an ultrasonic wave must be applied at some timing, in contrast to a detachment method by using a proteolytic enzyme represented by trypsin. In this regard, when a treatment time of the cell by using a cell detachment liquid increase, the cell may be damaged to cause a problem relating to cell culture thereafter. Therefore, it is necessary that the cell detachment step by using an ultrasonic wave is performed at an appropriate timing. However, regarding the adherent cell detachment method by using an ultrasonic wave, an ultrasonic application determination method to determine whether an ultrasonic wave is applied has not been found up to now.

That is, regarding the adherent cell detachment method by using an ultrasonic wave in the related art, for the above-described reason, there is a concern that cell detachment is not limited to being stable when a cell or a culture condition is changed.

A cell detachment method disclosed in the present specification is a cell detachment method for detaching a cell from a substrate by applying vibration to the cell adherent to the substrate, the method including an incubation step of incubating the cell after bringing a cell detachment liquid containing substantially no proteolytic enzyme into contact with the cell, an information acquisition step of acquiring information relating to the incubated cell, a determination step of determining a time when vibration is applied to the incubated cell in accordance with the information relating to the cell, and a detachment step of applying vibration to the incubated cell.

A cell detachment system disclosed in the present specification is a cell detachment system for detaching a cell from a substrate by applying vibration to the cell adherent to the substrate, the system including an incubation portion to incubate the cell after bringing a cell detachment liquid containing substantially no proteolytic enzyme into contact with the cell, an information acquisition portion to acquire information relating to the incubated cell, a determination portion to determine a time when vibration is applied to the incubated cell in accordance with the information relating to the cell, and a detachment portion to apply vibration to the incubated cell.

An information processing device disclosed in the present specification is an information processing device for controlling a cell detachment device to detach a cell from a substrate by applying vibration to the cell adherent to the substrate, the information processing device including an information acquisition portion to acquire information relating to the cell in contact with a cell detachment liquid containing substantially no proteolytic enzyme and a determination portion to determine a time when vibration is applied to the cell in accordance with the information relating to the cell.

Advantageous Effects of Invention

According to the present invention, a cell detachment method in which a cell survival rate is high, and a cell detachment efficiency is stable regardless of a change in a cell or a culture condition can be provided.

Brief Description of Drawings

[Fig. 1] Fig. 1 is a flow chart illustrating a cell detachment method according to an embodiment of the present invention.
[Fig. 2] Fig. 2 is a flow chart illustrating an example of the cell detachment method according to the embodiment of the present invention.
[Fig. 3] Fig. 3 is a flow chart illustrating another example of the cell detachment method according to the embodiment of the present invention.
[Fig. 4] Fig. 4 is a flow chart illustrating another example of the cell detachment method according to the embodiment of the present invention.
[Fig. 5] Fig. 5 is a diagram illustrating a configuration of an information processing device capable of executing a program according to the embodiment of the present invention.
[Fig. 6] Fig. 6 is a schematic diagram illustrating a cell detachment device according to the embodiment of the present invention.
[Fig. 7] Fig. 7 is a diagram illustrating a configuration of a cell detachment system according to the embodiment of the present invention.

Description of Embodiments

Regarding the method for detaching and recovering a cell from a substrate by using an ultrasonic wave without using a proteolytic enzyme, the present inventors performed intensive investigation on an ultrasonic application determination method for determining whether an ultrasonic wave is applied. As a result, it was found that, when a cell is incubated with a cell detachment liquid containing no degrading enzyme, a change in the area of a cell in a state of adhering to a substrate or a change in the brightness value of a cell image has relation to a cell detachment efficiency by using an ultrasonic wave thereafter and a survival rate of the detached cell.

In the cell detachment method according to the embodiment of the present invention, the cell is incubated with a cell detachment liquid containing substantially no degrading enzyme, and the cell information of the cell in a state of adhering to the substrate is measured in accordance with the newly obtained finding. Thereafter, in the cell detachment method according to the embodiment of the present invention, when the cell information (for example, area, brightness, and the like) are more than the threshold value, an ultrasonic wave is applied to the adherent cell. Consequently, a cell detachment method and a cell detachment device capable of minimizing damage to the cell and stably realizing a high detachment efficiency can be provided. As a result, a cell detachment method and a cell detachment device which are capable of automating cell detachment and obtaining a cell having stable quality can be provided.

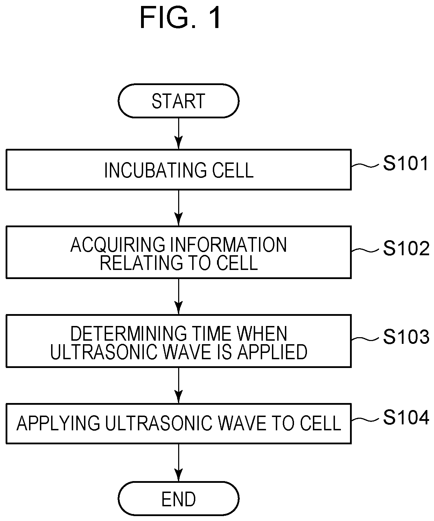

Since the present invention deals with the cell information where the cell is in an adherent state, the determination method essentially differs from the technique used for the purpose of determining "whether the cell is detached" in the above-described related art. The cell detachment method according to the embodiment of the present invention will be described below with reference to Fig. 1.

(Cell detachment method)

The cell detachment method according to the embodiment of the present invention is a cell detachment method in which the cell incubated on the substrate is detached from the substrate by applying vibration. The applied vibration may be vibration in an ultrasonic band. An embodiment will be described below with reference to an example in which vibration in an ultrasonic band is applied. The cell detachment method according to the present embodiment includes at least the following steps.

An incubation step of incubating the cell after bringing the cell (the cell adherent to the substrate) into contact with a cell detachment liquid containing substantially no proteolytic enzyme (S101).

An information acquisition step of acquiring information relating to the incubated cell (S102).

A determination step of determining a time when vibration is applied (an ultrasonic wave is applied) to the incubated cell in accordance with the information relating to the cell (S103).

A detachment step of applying vibration (applying an ultrasonic wave) to the incubated cell in accordance with the determined time (S104).

Herein, the incubation step denotes a step including at least one of a step of adjusting temperature, a step of adjusting humidity, and a step of adjusting an air composition. The step of adjusting temperature is, for example, a step of controlling a heater to set the temperature around a cell culture container to be about 37°C.

The step of adjusting humidity is, for example, a step of adjusting the humidity to 90% or more by disposing a humidifying vat to prevent a medium from drying. The step of adjusting an air composition is, for example a step of supplying $CO_2$ to set a $CO_2$ partial pressure to be about 5% to prevent the medium from oxidizing.

Herein, containing substantially no proteolytic enzyme means that an amount of the proteolytic enzyme relative to a total amount of the cell detachment liquid is 0.0005% by mass or less.

In this regard, the cell detachment liquid may contain a metal ion chelating agent, and the cell detachment liquid may contain polyethylene glycol.

It is preferable that the incubation step be performed in an environment of 37°C.

In this regard, the information acquisition step S102 may include a step of acquiring an image of the cell, and the information relating to the cell may be at least one of an area of a region of the cell, an area of a halo region of the cell, and a brightness value of the region of the cell in the image of the cell.

(Cell)

There is no limitation regarding the cell according to the present embodiment provided that the cell can be cultured while adhering to culture instruments in vitro. Examples include Chinese hamster ovary CHO cells, various cultured cell lines, such as connective mouse tissue L929 cells, mouse skeletal muscle myoblast cells (C2C12 cells), human fetal lung normal diploid fibroblast cells (TIG-3 cells), human embryonic kidney cells (HEK293 cells), adenocarcinomic human alveolar basal epithelial A549 cells, and human cervical cancer Hela cells. In addition, examples include epithelial cells and endothelial cells constituting each tissue and organ in a living body, contractile skeletal muscle cells, smooth muscle cells, cardiac muscle cells, neutron cells constituting the nervous system, glia cells, fibroblast cells, hepatic parenchymal cells, non-parenchymal cells, and adipose cells involve in living body metabolism, cells having differentiation potency, such as various stem cells, more specifically, induced pluripotent stem (iPS) cells, embryonic stem (ES) cells, embryonic germ (EG) cells, embryonic cancer (EC) cells, mesenchymal stem cells, hepatic stem cells, pancreatic stem cells, skin stem cells, muscle stem cells, and germ stem cells, or precursor cells of each tissue and cells differentiation-induced therefrom. The cell detachment method according to the present embodiment is suitable for cells having a strong intercellular bond, cells having strong adhesion to the substrate, and cells having high trypsin sensitivity of these cells. Due to needs for mass culture of cells, the cell detachment method is particularly suitable for, for example, CHO cells used for protein production and mesenchymal stem cells usable for cell therapy.

(Substrate)

The substrate according to the present embodiment means a culture container used for cell culture. There is no limitation regarding the culture container provided that the culture container has adhesiveness to the cell, and examples include flasks, tissue culture flasks, dishes, Petri dishes, tissue culture dishes, multi-dishes, microplates, multi-well plates, multi-plates, Shales, culture bags, and bottles.

It is sufficient that the material for forming the substrate according to the present embodiment is a material that is chemically stable and that can culture a predetermined cell, and examples include polyethylene, polypropylene, polycarbonate, polystyrene, polyvinyl chloride, nylon, polyurethane, polyurea, polylactic acid, polyglycolic acid, polyvinyl alcohol, polyvinyl acetate, poly(meth)acrylic acid, poly(meth)acrylic acid derivatives, polyacrylonitrile, poly(meth)acrylic amide, poly(meth)acrylic amide derivatives, polysulfone, cellulose, cellulose derivatives, polysilicone, polymethylpentene, glass, and metal. Of these, polystyrene is preferable.

(Cell detachment liquid)

The cell detachment liquid according to the embodiment of the present invention is a solution containing substantially no proteolytic enzyme and is a solution used in the cell detachment method according to the present embodiment to detach the cell. In other words, the cell detachment liquid is a solution for decreasing the adhesion of the cell.

There is no limitation regarding the cell detachment liquid according to the embodiment of the present invention provided that the cell detachment liquid is an aqueous solution capable of culturing the cell (also referred to as medium), and various salts and factors may be contained. There is no limitation regarding the factor contained in the medium, and proteins, such as albumin, insulin, and transferrin, selenium, antibiotics, saccharide such as glucose, lipid, and synthetic polymers may be contained.

The pH of the cell detachment liquid according to the present embodiment is preferably in a neutral region. Herein, the neutral region is set to have a pH of 6 or more and pH of 8 or less. The reason for this is that the neutral region is suitable for cell culture and can stably maintain a high cell survival rate. The pH can be appropriately adjusted by using hydrochloric acid, sodium hydroxide, and the like. In this regard, to stably maintain the pH, various buffer solutions are preferably used.

The viscosity of the cell detachment liquid according to the present embodiment is preferably 1.80 mPa·s or less. The reason for this is that the flow of the detachment liquid generated by ultrasonic vibration is not hindered and a high detachment efficiency can be maintained. The viscosity of the cell detachment liquid can be appropriately adjusted by, for example, addition of a polymer or saccharide.

Regarding the cell detachment liquid according to the present embodiment, an amount of proteolytic enzyme relative to a total amount of the cell detachment liquid is preferably 0.0005% by mass or less, and more preferably, the proteolytic enzyme is not added. The reason for this is that, as described in Background Art, since the proteolytic enzyme decomposes a portion of the cell, the detachment efficiency can be increased, but, at the same time, the quality of the cell may deteriorate.

The proteolytic enzyme according to the present embodiment is an enzyme for, for example, decomposing a portion of the cell to readily detach the cell from the substrate. Examples include trypsin, accutase, collagenase, natural protease, chymotrypsin, elastase, papain, and pronase or recombinants thereof.

A solution containing a metal ion chelating agent (hereafter also referred to as chelating agent) is particularly preferable as the cell detachment liquid according to the present embodiment. The reason for this is that using the cell detachment liquid containing the chelating agent enables the cell to be efficiently detached by ultrasonic vibration.

There is no limitation regarding the chelating agent according to the present embodiment, and examples include ethylenediaminetetraacetic acid (hereafter also referred to as EDTA), ethylenediamine, ethylenediamine tetra(methylenephosphonic acid), glycol ether diaminetetraacetic acid, nitriltriacetic acid, diethylenetriaminepentaacetic acid, iminodiacetic acid, dihydroxyethylglycine, dicarboxymethylglutamic acid, ethylenediaminedisuccinic acid, etidronic acid, citric acid, gluconic acid, and phosphonobutanetriacetic acid. Of these, a chelating agent that forms a chelate with a divalent cation is preferable, a chelating agent that forms a chelate with $Ca^{2+}$ and $Mg^{2+}$ is particularly preferable, and ethylenediaminetetraacetic acid is most preferable. When ethylenediaminetetraacetic acid is used as the chelating agent, the pH of the cell detachment liquid is preferably 7.0 or more and 8.0 or less. The reason for this is that the pH in the neutral region in which a high cell survival rate can be maintained being set to be somewhat high enables the chelate ability of ethylenediaminetetraacetic acid to be enhanced and enables the detachment efficiency to be increased. In this regard, the chelating agents may be used alone, or two or more types thereof may be used in combination.

The content of the chelating agent is preferably 0.01 mM or more and 5.0 mM or less. Setting the content to be within this range enables the chelate effect to be reliably obtained and enables the activity of the cell to be suppressed from deteriorating due to the presence of an excessive chelating agent.

The cell detachment liquid according to the present embodiment may contain a hydrophilic polymer having a polyalkylene glycol structure. The polyalkylene glycol can increase the cell survival rate in the cell detachment method by using an ultrasonic wave. Examples of the hydrophilic polymer having a polyalkylene glycol structure include polyethylene glycols. The hydrophilic polymer has a peak molecular weight Mp measured by gel permeation chromatography of preferably 800 or more and 50,000 or less and further preferably 1,200 or more and 20,000 or less. The reason for this is that the influence of the polymer on the cell is low and that a medium thickening effect of the polymer can be suppressed.

(Buffer solution)

The buffer solution according to the present embodiment can be used without limitation provided that the neutral region can be maintained. Herein, the neutral region is set to have a pH of 6 or more and a pH of 8 or less. Examples include Tris buffer solutions such as Tris-HCl buffer solutions, phosphate buffer solutions, HEPES buffer solutions, citrate-phosphate buffer solutions, glycylglycine-sodium hydroxide buffer solutions, Britton-Robinson buffer solutions, and GTA buffer solutions. Of these, phosphate buffer solutions close to the environment in a living body are preferable, and phosphate buffered saline (PBS) adjusted to be isotonic to the liquid in the cell is more suitable for use as the phosphate buffer solution.

(Medium)

There is no limitation regarding the type of the medium, and examples include Dulbecco's Modified Eagles's Medium (DMEM), Ham's F12 Medium (Ham's Nutrient Mixture F12), DMEM/F12 Medium, McCoy's 5A medium, Eagle's Minimum Essential Medium (EMEM), alpha Modified Eagle's Minimum Essential Medium ($\alpha$MEM), MEM Medium (Minimum Essential Medium), RPMI1640 Medium, Iscove's Modified Dulbecco's Medium (IMDM), MCDB131 Medium, William's Medium E, IPL41 Medium, Fischer's Medium, StemSpan H3000 (produced by STEMCELL Technologies), StemSpanSFEM (produced by STEMCELL Technologies), Stemlinell (produced by Sigma-Aldrich), Endothelial Cell Growth Medium 2 Kit (produced by PromoCell), Mesenchymal Stem Cell Growth Medium 2 (produced by PromoCell), MSCGM Bullet Kit (produced by Lonza), mTeSR1 or 2 Medium (produced by STEMCELL Technologies), ReproFF or ReproFF2 (produced by REPROCELL), NutriStem Medium (produced by Biological Industries), and MF-Medium mesenchymal stem cell growth medium (produced by Toyobo Co., Ltd.).

Of these, it is preferable that a medium suitable for culture of each cell be used.

(Serum)

A serum or an antibiotic may be added to the above-described medium. Examples of the serum include Fetal Bovine Serum (FBS), Calf Serum, Adult Bovine serum, Horse Serum, Sheep Serum, Goat Serum, Porcine Serum, Chicken Serum, Rabbit Serum, and Human Serum, and in general, FBS is frequently used due to ease of availability. In this regard, the medium may be a serum-free medium containing neither untreated nor unrefined serum and containing a component derived from serum or a compound derived from animal tissue (growth factor or the like).

(Antibiotic)

Examples of the antibiotic include penicillin, streptomycin, ampicillin, carbenicillin, tetracycline, bleomycin, actinomycin, kanamycin, actinomycin D, and amphotericin B.

(Cell culture condition)

The cell culture condition can be appropriately selected in accordance with the cell to be cultured. In general, an appropriate medium is added to a dish, about $1.0 \times 10^1$ to $5.0 \times 10^4$ cells/cm$^2$ of cells are seeded thereon, and culture is performed in an environment of 37°C and a $CO_2$ concentration of 5%. In such an instance, it is preferable that the culture be performed until the cell occupation area rate in the substrate reaches about 70% to 80%, that is, a so-called subconfluent state.

(Cell detachment method by using ultrasonic wave)

The cell detachment method according to the embodiment of the present invention is a detachment method, by using an ultrasonic wave, for detaching an adherent cell cultured on the substrate, the method including Steps [1] to [4] below.

[1] A step of bringing a cell in a state of adhering to the substrate into contact with a cell detachment liquid.
[2] A step of performing incubation.
[3] A step of measuring cell information of the cell in the state of adhering to the substrate.
[4] A step of detaching the cell from the substrate by applying an ultrasonic wave to the adherent cell when the cell information exceeds the threshold value.

Each step will be described below.

(Step [1])

Regarding Step [1] in the cell detachment method according to the embodiment of the present invention, a cell in a state of adhering to the substrate is brought into contact with a cell detachment liquid containing substantially no proteolytic enzyme. Step [1] is a step of exchanging a growth medium in a cell culture state for a detachment liquid and means an operation of medium exchange.

The cell may be washed with a buffer solution before or after Step [1]. For example, when it is intended to detach the cultured cell from the substrate, an effect of the cell detachment liquid may be enhanced by removing the growth medium at first, and washing the cell in the state of adhering to the substrate with the buffer solution before the cell detachment liquid is added. The reason for this is that, in general, a factor such as a serum which hinders cell detachment is contained in a growth medium.

(Step [2])

Regarding Step [2], incubation is performed while the cell in the state of adhering to the substrate is in contact with the cell detachment liquid containing substantially no proteolytic enzyme. "Performing incubation" denotes performing a step including at least one of a step of adjusting temperature, a step of adjusting humidity, and a step of adjusting an air composition. The step of adjusting temperature is, for example, a step of controlling a heater to set the temperature around a cell culture container to be about 37°C. The step of adjusting humidity is, for example, a step of adjusting the humidity to 90% or more by disposing a humidifying vat to prevent the medium from drying. The step of adjusting an air composition is, for example, a step of supplying $CO_2$ to set a $CO_2$ partial pressure to be about 5% to prevent the medium from oxidizing.

As described later, it was found that a contact time between the cell and the cell detachment liquid in Step [2] is very important for the detachment efficiency and the cell survival rate. Since the incubation time is changed in accordance with the type of the cell and the culture condition, as described later, it is necessary to determine the timing of application of ultrasonic vibration by measuring the cell information in a later step. There is no limitation regarding the incubation time, and the incubation time is within the range of, for example, 30 sec to 20 min.

There is no limitation regarding the environmental temperature of Step [2]. From the viewpoint of maintaining the cell survival rate, 10°C to 40°C is preferable, and 30°C to 40°C is a temperature most suitable for growing the cell and is particularly preferable.

According to Step [2], the adhesiveness to the substrate of the cell adherent to the substrate is lowered, and detachment by an ultrasonic wave thereafter is made to be possible. In other words, it is difficult to detach the cell at a high detachment efficiency without performing Step [1] and Step [2].

As described later with reference to a flow of cell detachment, incubation is performed in Step [2], and movement to measurement of the cell information in Step [3] is appropriately performed. Thereafter, it is preferable that a loop in which, when movement to application of the ultrasonic wave in Step [4] is not performed, return to Step [2] is performed to restart incubation be repeated.

(Step [3])

Regarding Step [3], the information of the cell that is in the state of adhering to the substrate and that is incubated in the cell detachment liquid containing substantially no degrading enzyme is measured.

Step [3] (measurement of cell information) may be performed before Step [2] above.

In addition, Step [3] includes a process to determine whether movement to next Step [4] is performed, as described below.

In this regard, it is sufficient that the process to determine whether movement to Step [4] is performed is a step of determining a time when the detachment step of detaching the cell is started. Herein, the time may be an absolute time such as 13:30 or may be a relative time such as 30 sec after a point in time when the time is determined, a point in time when an incubation step is started, or a point in time when acquirement of the cell information is started. The time when the detachment step is stated may be immediately after a point in time when the time is determined.

Examples of the cell information include an area of the cell, an area of a halo region in a phase contrast microscope image (also referred to as phase contrast image), a brightness value in the phase contrast image, and a brightness value when a cell culture surface of the substrate is observed by oblique-incidence illumination.

Regarding an example of an area of the cell, digitization of an area of the cell can be performed by, for example, acquiring a phase contrast image of an adherent cell and measuring an occupied area of a cell by image processing.

It is known that an adherent cell comes out having a large area in a phase contrast image, and a cell having a lowered adhesion due to an action of the cell detachment liquid comes out having a small cell area in a phase contrast image since the cell takes on a shape close to a sphere due to the surface tension. This phenomenon is utilized, the area of the cell in the phase contrast image is digitized, and a change thereof is observed. As described later in Examples, in accordance with the type of the cell and the culture condition, the cell can be effectively detached by performing movement to next (Step [4]) at some point in time of the change in the area of the cell, for example, at a point in time when the cell area change rate becomes constant, to apply an ultrasonic wave. That is, the above-described point in time when the cell area change rate becomes constant is a timing most suitable for applying an ultrasonic wave to detach an adherent cell cultured on the substrate, and movement to next (Step [4]) can be determined.

Examples of the other cell information include a brightness value in the phase contrast image or an area of a halo region.

Regarding the phase contrast image of the cell in the adherent state, only a region with a small phase difference (cell is a flat state) is detected. On the other hand, it is known that adhesion of the cell is weakened by adding the detachment liquid, the phase difference around the cell lifted from the substrate is increased, and a halo region having a brightness value higher than the other is observed. That is, the area of the halo region increases with weakening adhesion of the cell, and the value of the area becomes nearly constant. For example, it is sufficient that the halo region is measured, and an ultrasonic wave is applied at a point in time when the area of the halo region becomes largest. Alternatively, the brightness value of the image is measured, and an ultrasonic wave may be applied at a point in time when the brightness value of the image is brightest.

Herein, the halo region will be further described. When a cell is observed by using a phase contrast microscope, a contour of light called halo appears between the cell and a region in which the cell is not present. Regarding the phase contrast observation, the halo has relatively high brightness and is an artifact. However, as described above, the halo has relation to an adhesion state of the cell and, therefore, is useful as the cell information.

Digitation of the brightness value or the area of the halo region in the phase contrast image can be performed by image processing software, for example, ImageJ (National Institutes of Health, NIH).

An example of treatment will be described. Initially, a bright field image of the cell is picked up by using a phase contrast microscope. Subsequently, the image file is taken into ImageJ. The image is converted to an 8-bit grayscale image. Herein, the range from the maximum value to the minimum value of the brightness in the image is prorated 0 to 255. Next, a region of interest may be selected. Regarding the ImageJ, a plurality of objects in the image can be selected by using the ROI (Region of Interest) Manager. Next, Measure is executed. The brightness value obtained here is a brightness value per unit pixel or a value of product of the brightness value of each pixel times the area of the object. Integrated Density (total sum of brightness) can digitize the brightness value of the image. An example of the brightness value according to the embodiment of the present invention is Integrated Density (hereafter simply referred to as brightness value). The brightness value is an average relative value, and regarding the unit, a dimensionless quantity, the number of pixels, or the like is appropriately used (in accordance with a digitization method).

The brightness value of the phase contrast image in Example described below is expressed by the product of the area of the measurement object times the average brightness. Only the brightness value of the measurement object can be determined by appropriately setting Threshold (threshold value) to perform division into the measurement object and Background (background).

An example of a method for measuring the area of the halo region will be described. Regarding the 8-bit grayscale image, Threshold (threshold value) is set. Herein, division into the halo region and Background (background) is performed. The halo region may be visually extracted, or the halo region may be automatically extracted by designating various threshold value setting methods. Subsequently, the area rate of the extracted halo region is determined. The area rate of the extracted halo region can be digitized as %Area by executing Measure (measurement).

In this regard, the brightness value when the cell culture surface of the substrate is observed by oblique-incidence illumination is mentioned as another cell information. Regarding the cell having a lowered adhesion due to an action of the cell detachment liquid, since a pseudopodium is shrunk and a microscopic surface adhering to the substrate is decreased, light tends to scatter at the interface between the substrate and the cell. Consequently, when the observation is performed by using oblique-incidence illumination which increases the ratio of scattered light, the brightness value of the cell culture surface of the substrate is increased with weakening adherent state of the cell so that the value becomes nearly constant. Therefore, the brightness value of the cell culture surface of the substrate when observation is made by oblique-incidence illumination may be measured, and an ultrasonic wave may be applied at a point in time when the brightness value becomes constant.

As described later in Example, in accordance with the type of the cell and the culture condition, the cell can be effectively detached by performing movement to next (Step [4]) at a characteristic point in time of the change in the measured cell information, for example, at a point in time when the cell area change rate becomes constant, to apply an ultrasonic wave. That is, the above-described point in time when the cell area change rate becomes constant is a timing most suitable for applying an ultrasonic wave to detach an adherent cell cultured on the substrate, and movement to next (Step [4]) can be determined.

Determining the threshold value of the change rate of the cell in advance in accordance with the type of the cell and the culture condition readily automates the cell detachment. That is, the embodiment according to the present invention includes an ultrasonic application timing determination method for measuring the cell information of the cell and performing movement to next (Step [4]) of applying an ultrasonic wave to the adherent cell when the cell information is more than the threshold value and an ultrasonic application determination device to execute the method.

The cell information can adopt various parameters. Examples include an area of each cell, a size of each cell, a roundness of each cell, a light transmittance of each cell, an occupied area of a cell group, a brightness value of a cell image, an area in which no cell is present, an area of a halo region, a brightness value of an image based on the halo, and a brightness value of an image when a cell culture surface of a substrate is observed by oblique-incidence illumination.

The cell information can be measured by various methods. For example, acquiring a phase contrast image of the cell by using a phase contrast microscope and analyzing the resulting image enable various cell information above to be measured. In addition, the brightness value of the substrate culture surface can be measured by using an illumination means to perform oblique-incidence illumination with respect to the cell culture surface of the substrate and an observation means disposed in the direction perpendicular to the culture surface. In this regard, the illumination means, and the observation means may be disposed at positions opposite each other with the substrate interposed therebetween or may be disposed in the same direction provided that the positions readily receive the light scattered at the cell culture surface. Further, using ring illumination as the illumination means enables brightness variations in the culture surface to be decreased.

(Calculation example of cell area as cell information)

Phase contrast images of the cell were acquired over time from immediately after exchange to the cell detachment liquid. Marking of a specific cell was performed, and the area of the cell was quantified by image processing software. The area of the cell immediately after coming into contact with the cell detachment liquid (incubation time of zero) is denoted by A1. The area of the cell with an incubation time t in the cell detachment liquid is denoted by A2.

The thus determined cell area decreases over time with weakening adhesion of the cell to the substrate. For example, the cell area that was 237 square micrometers decreased to 113 square micrometers due to incubation for 10 min. When incubation was continued, change was stopped at about 100 square micrometers. Therefore, movement to Step [4] of performing ultrasonic application can be determined at a point in time when the cell area reaches about 100 square micrometers.

(Calculation example of cell area change rate as cell information)

The phase contrast images of the cell were acquired over time from immediately after exchange to the cell detachment liquid. Marking of a specific cell was performed, and the area of the cell was quantified by image processing software. The area of the cell immediately after coming into contact with the cell detachment liquid (incubation time of zero) is denoted by A1. The area of the cell with an incubation time t in the cell detachment liquid is denoted by A2. The cell area change rate is determined by Formula (I) below.

$$(A1 - A2)/A1 \quad (I)$$

The thus determined cell area change rate increases over time with weakening adhesion of the cell to the substrate. For example, the change rate was 0.15 due to incubation for 1 min but became constant at about 0.5 due to incubation for 5 min. Therefore, movement to Step [4] of performing ultrasonic application can be determined at a point in time when 0.5 is reached.

The cell information can be set as the threshold value in advance in accordance with the cell species and the culture condition. For example, in the above-described example, the threshold value of the cell area change rate is set to be 0.5.

Regarding the cell detachment, in Step [3], the cell information is measured, and movement to Step [4] can be determined at a point in time when 0.5 is reached.

(Calculation example of halo region area rate as cell information)

The phase contrast images of the cell were acquired over time from immediately after exchange to the cell detachment liquid. The halo region area rate was measured by appropriately performing binarization, setting of the threshold value, and area measurement of these phase contrast images of the cell by the image processing software. In general, the brightness value depends on a cell information acquisition means and is an average relative value, and regarding the unit, a dimensionless quantity, the number of pixels, or the like is appropriately used. For example, the brightness value is a brightness value per unit pixel or a value of product of the brightness value of each pixel times the area of the object. Regarding measurement of the brightness value, digitization can be performed by using various image processing software. For example, digitization can be performed by using the image processing software such as ImageJ (National Institutes of Health, NIH).

The thus determined halo region area rate increases over time with weakening adhesion of the cell to the substrate. For example, the halo region area rate of the phase contrast image when the incubation time in the cell detachment liquid was 30 sec was 2.8%. The halo region area rate of the phase contrast image when the incubation time in the cell detachment liquid was 5 min was 49.9%. Even when the incubation time was further increased, the halo region area rate was not increased. Therefore, movement to Step [4] of performing ultrasonic application can be determined at a point in time when the halo region area rate reaches 49.9%.

(Calculation example of brightness value of phase contrast image as cell information)

The phase contrast images of the cell were acquired over time from immediately after exchange to the cell detachment liquid.

The brightness value of the phase contrast image was measured by performing binarization, setting of the threshold value, and brightness measurement of these phase contrast images of the cell by the image processing software.

The thus determined brightness value of the phase contrast image increases over time with weakening adhesion of the cell to the substrate since the halo region increases. For example, the brightness value of the phase contrast image when the incubation time in the cell detachment liquid was 30 sec was 7.2. The brightness value of the phase contrast image when the incubation time in the cell detachment liquid was 5 min was 127.2. Even when the incubation time was further increased, the brightness value of the phase contrast image was not increased. Therefore, movement to Step [4] of performing ultrasonic application can be determined at a point in time when the brightness value of the phase contrast image reaches 127.2.

(Calculation example of brightness value of oblique-incidence illumination observation image as cell information)

The observation images of the cell culture surface subjected to oblique-incidence illumination were acquired over time from immediately after exchange to the cell detachment liquid. The brightness value was measured by performing brightness measurement of the entire cell culture surface of the substrate by the image processing software.

The thus determined brightness value of the cell culture surface observed by oblique-incidence illumination increases over time with weakening adhesion of the cell to the substrate since the scattered light intensity increases. For example, an average value of the brightness of the entire culture surface of the substrate when the incubation time in the cell detachment liquid was 30 sec was 96.2. An average value of the brightness of the entire culture surface of the substrate when the incubation time in the cell detachment liquid was 5 min was 108.0. Even when the incubation time was further increased, an average value of the brightness of the entire culture surface was almost not increased. Therefore, movement to Step [4] of performing ultrasonic application can be determined at a point in time when an average value of the brightness of the culture surface of the substrate in the scattered light observation image reaches 108.0.

(Step [4])

Regarding Step [4], the cell is detached from the substrate by applying an ultrasonic wave to the adherent cell.

(Vibration due to ultrasonic wave)

Vibration due to an ultrasonic wave according to the present embodiment can be exemplified with vibration having a frequency of about 10 kHz to 1 MHz as described in International Publication No. 2016/047368. There is no limitation regarding a usable vibration generation means provided that the means can apply vibration due to an ultrasonic wave to the cell. Examples include an ultrasonic transducer such as lead zirconate titanate (PZT) as described in Japanese Patent Laid-Open No. 2008-92857.

Herein, as described in Japanese Patent Laid-Open No. 2006-314204, vibration can be applied to a culture container by a vibrator being brought into direct contact with an outer surface of the culture container filled with a medium and sealed. In addition, as described in International Publication No. 2016/047368, an ultrasonic wave can be incident on the cell that is a detachment object by interposing an ultrasonic wave-transmitting substance between an ultrasonic application means and a treatment object region rather than bringing the ultrasonic application means into direct contact with the container.

(Cell detachment by applying vibration due to ultrasonic wave)

Regarding Step [4], the cell is detached from the substrate by applying vibration due to an ultrasonic wave to the cell.

Step [4] of the cell detachment method according to the present embodiment may include a step of applying an external stimulus such as convection to the culture solution in addition to the above-described step of applying vibration due to an ultrasonic wave. Examples of the method for generating convection to the culture solution include pipetting and using a pump or a mixing blade.

There is no limitation regarding the time to apply vibration due to an ultrasonic wave in Step [4] above, and, the time is, for example, 1 sec to 1 hour, preferably 5 sec to 30 min, and further preferably 10 sec to 15 min from the viewpoint of effectively detaching the cell and increasing the cell survival rate.

There is no limitation regarding the environmental temperature in Step [4] above, and the temperature is preferably 20°C to 40°C, particularly preferably 30.0°C or higher and 37.5°C or lower from the viewpoint of increasing the cell survival rate. The reason for this is that the temperature during cell detachment is close to the temperature during culture, an influence of a temperature change on the cell can be decreased, and a high survival rate can be maintained.

(Vibration due to ultrasonic wave)

(Cell detachment flow 1)

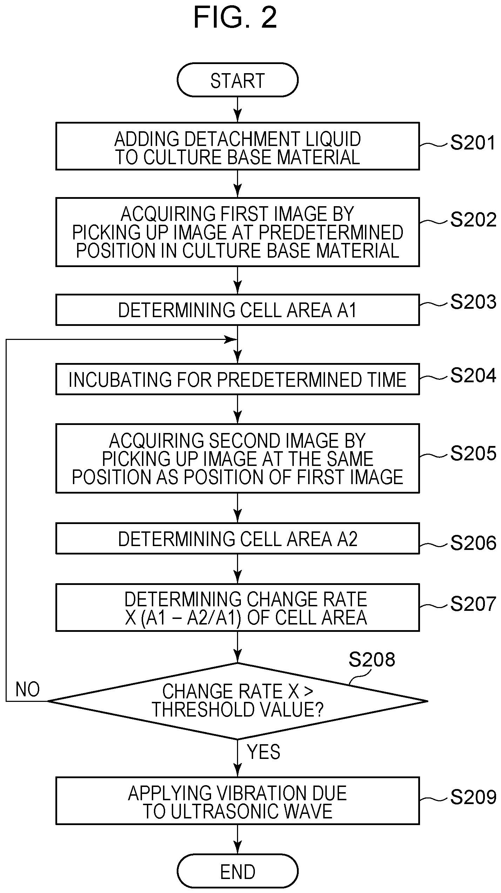

An example of the cell detachment method according to an embodiment of the present invention is performed in accordance with a flow chart illustrated in Fig. 2, as described below.

Initially, as Step [1], the cell detachment liquid is added to the culture substrate (S201). Thereafter, the culture substrate is transferred to a cell information acquisition portion (image acquisition portion or the like) by a manual operation, a robot operation, or the like. In the cell information acquisition portion, a first image is acquired by picking up an image (phase contrast image or the like) at a predetermined position in the culture substrate (S202). A cell area A1 is determined from the first image (S203). Herein, an image processing device may be used. In this regard, Step [3] (measurement of cell information) is performed before Step [2], but there is no problem.

Next, as Step [2], a step of incubating for a predetermined time is performed (S204).

Subsequently, as Step [3], after a lapse of a predetermined time during incubation, a second image is acquired by picking up an image at the same position as the position of the first image in the manner akin to that of S202 (S205). A cell area A2 is determined from the second image (S206).

Next, a change rate X ($(A1 - A2)/A1$) of the cell area is determined by an operator or an image processing device (S207). The resulting change rate X is transferred to a check by an operator or an ultrasonic application determination device, and the operator or the ultrasonic application determination device compares the change rate X with a threshold value set in advance (S208). When the change rate X is smaller than the threshold value, the treatment is returned to S204, and S204 to S208 are repeated. When the change rate X is larger than or equal to the threshold value, the culture substrate is transferred to an ultrasonic application portion, and an ultrasonic wave is applied there to the culture substrate (S209). That is, Step [4] is performed. Thereafter, a cell suspension liquid containing a detached cell is recovered to complete cell detachment.

(Cell detachment flow 2)

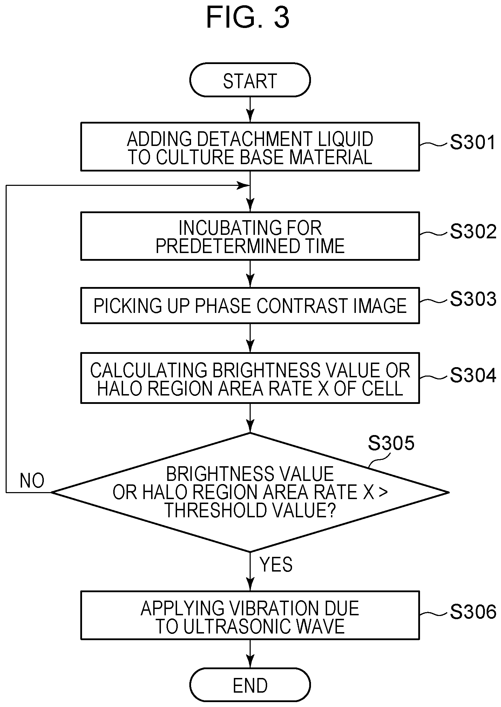

An example of the cell detachment method according to an embodiment of the present invention is performed in accordance with a flow chart illustrated in Fig. 3, as described below.

Initially, as Step [1], the cell detachment liquid is added to the culture substrate (S301). Next, as Step [2], a step of incubating for a predetermined time is performed (S302).

Subsequently, as Step [3], after a lapse of a predetermined time during incubation, a first image is acquired by picking up a phase contrast image of a cell at a predetermined position in the culture substrate by using a phase contrast microscope (S303). The brightness value of the cell or the halo region area rate is calculated from the first image (S304). Next, the calculated brightness value of the cell or the halo region area rate X is compared with a threshold value set in advance (S305). When X is smaller than the threshold value, the treatment is returned to S301, and S3010, and S303 to S305 are repeated. When X is larger than or equal to the threshold value, the culture substrate is transferred to an ultrasonic application portion, and an ultrasonic wave is applied to the culture substrate (S306). That is, Step [4] is performed. Thereafter, a cell suspension liquid containing a detached cell is recovered to complete cell detachment.

(Cell detachment flow 3)

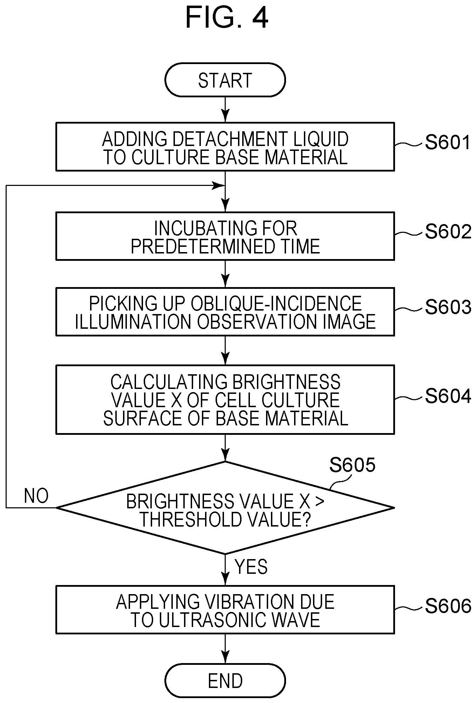

An example of the cell detachment method according to an embodiment of the present invention is performed as described below in accordance with a flow chart illustrated in Fig. 4.

Initially, as Step [1], the cell detachment liquid is added to the culture substrate (S601). Next, as Step [2], a step of incubating for a predetermined time is performed (S602).

Subsequently, as Step [3], after a lapse of a predetermined time during incubation, a first image is acquired by picking up an observation image of the cell culture surface subjected to oblique-incidence illumination (S603). An average value of the brightness of the culture surface is calculated from the first image (S604). Next, the calculated brightness value X is compared with a threshold value set in advance (S605). When X is smaller than the threshold value, the treatment is returned to S602, and S40, and S603 to S605 are repeated. When X is larger than or equal to the threshold value, the culture substrate is transferred to an ultrasonic application portion, and an ultrasonic wave is applied there to the culture substrate (S606). That is, Step [4] is performed. Thereafter, a cell suspension liquid containing a detached cell is recovered to complete cell detachment.

(Treatment after cell detachment)

After Step [4] of detaching the cell from the substrate by applying vibration due to an ultrasonic wave to the cell adherent to the substrate, the detached cell may be seeded in a culture container, such as a new substrate, a dish, and a flask, or may be moved to a step of performing cell marking or the like. Alternatively, the cell may be used as a sample for measuring the number of cells or the cell survival rate. Further, when the obtained cells associate to take on a state of a cell mass, single-cell preparation may be appropriately performed.

(Configuration of information processing system)

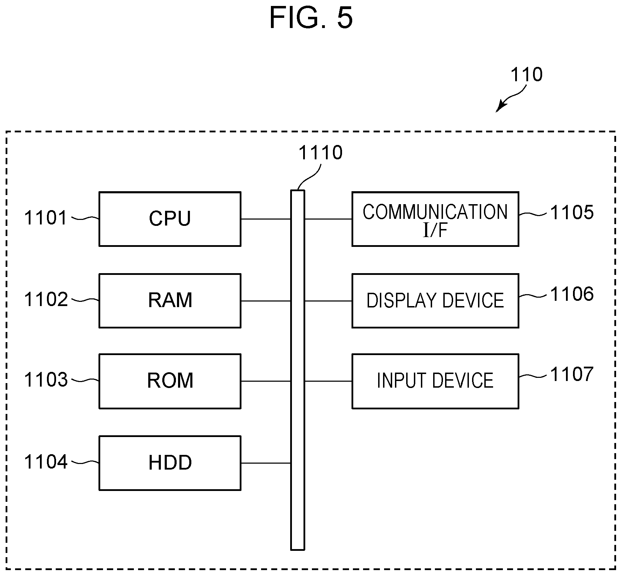

Fig. 5 is a block diagram illustrating a hardware configuration example of an information processing system 110 capable of executing a program according to the embodiment of the present invention. The information processing system 110 is an example of an information processing device and includes an information acquisition portion to acquire information relating to the cell in contact with the cell detachment liquid containing substantially no proteolytic enzyme and a determination portion to determine a time when vibration is applied to the cell in accordance with the information relating to the cell. The functions of the information acquisition portion and the determination portion are realized by, for example, CPU 1101.

The information processing system 110 has a function of a computer. For example, the information processing system 110 may be configured to be integrated with a desktop PC (Personal Computer), a laptop PC, a tablet PC, a smartphone, or the like.

To realize the function of the computer to perform computation and storage, the information processing system 110 includes CPU (Central Processing Unit) 1101, RAM (Random Access Memory) 1102, ROM (Read Only Memory) 1103, and HDD

(Hard Disk Drive) 1104. In addition, the information processing system 110 includes a communication I/F (interface) 1105, a display device 1106, and an input device 1107. CPU 1101, RAM 1102, ROM 1103, HDD 1104, the communication I/F 1105, the display device 1106, and the input device 1107 are connected to each other through a bus 1110. In this regard, the display device 1106 and the input device 1107 may be connected to the bus 1110 through a driving device, not illustrated in the drawing, to drive these devices.

In Fig. 5, the portions constituting the information processing system 110 are illustrated as an integrated device, but a portion of these functions may be configured as an external device. For example, the display device 1106 and the input device 1107 may be an external device other than a portion constituting the functions of the computer including CPU 1101 and the like.

CPU 1101 performs predetermined functions in accordance with the programs stored in RAM 1102, HDD 1104, and the like and has also a function of controlling each portion of the information processing system 110. RAM 1102 is formed from a volatile storage medium and provides a temporary memory region necessary for the operation of CPU 1101. ROM 1103 is formed from a nonvolatile storage medium and stores necessary information such as programs used for operation of the information processing system 110. HDD 1104 is formed from a nonvolatile storage medium and is a storage device to store the information relating to the number and the positions of individual independent separation blocks, fluorescence intensity, and the like.

The communication I/F 1105 is a communication interface in accordance with the standard of Wi-Fi (registered trademark), 4G, or the like and is a module to perform communication with other devices. The display device 1106 is a liquid crystal display, OLED (Organic Light Emitting Diode) display, or the like and is used for displaying videos, still images, characters, and the like. The input device 1107 is a button, a touch panel, a keyboard, a pointing device, or the like and is used by an operator to operate the information processing system 110. The display device 1106 and the input device 1107 may be integrally formed as a touch panel.

In this regard, the hardware configuration illustrated in Fig. 5 is an exemplification, devices other than these may be added, and a portion of devices are not limited to be disposed. In this regard, a portion of devices may be replaced with other devices having the same functions. Further, a portion of functions may be provided from other devices through a network, and the functions constituting the present embodiment may be dispersed to a plurality of devices to be realized. For example, HDD 1104 may be replaced with SSD (Solid State Drive) by using a semiconductor element such as flash memory or may be replaced with cloud storage.

(Cell detachment system)

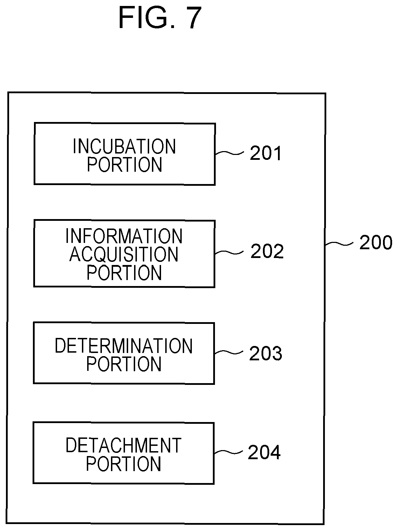

Fig. 7 is a diagram illustrating a configuration of a cell detachment system according to the embodiment of the present invention. A cell detachment system 200 includes an incubation portion 201, an information acquisition portion 202, a determination portion 203, and a detachment portion 204. The cell detachment system 200 may be a cell detachment device described later. The cell detachment system 200 may be formed from a plurality of devices, and the function of the cell detachment system 200 may be realized by the plurality of devices.

(Cell detachment device)

An example of the cell detachment device by using an ultrasonic wave according to the embodiment of the present invention is a cell detachment device to detach a cell cultured on the substrate and includes a medium exchange portion to bring the cell in a state of adhering to the substrate into contact with a cell detachment liquid. In addition, the cell detachment device is a cell detachment device including a cell information acquisition portion to acquire and measure the cell information of the cell, an ultrasonic application determination portion to determine an ultrasonic application timing, and an ultrasonic application portion to apply vibration due to an ultrasonic wave to the cell.

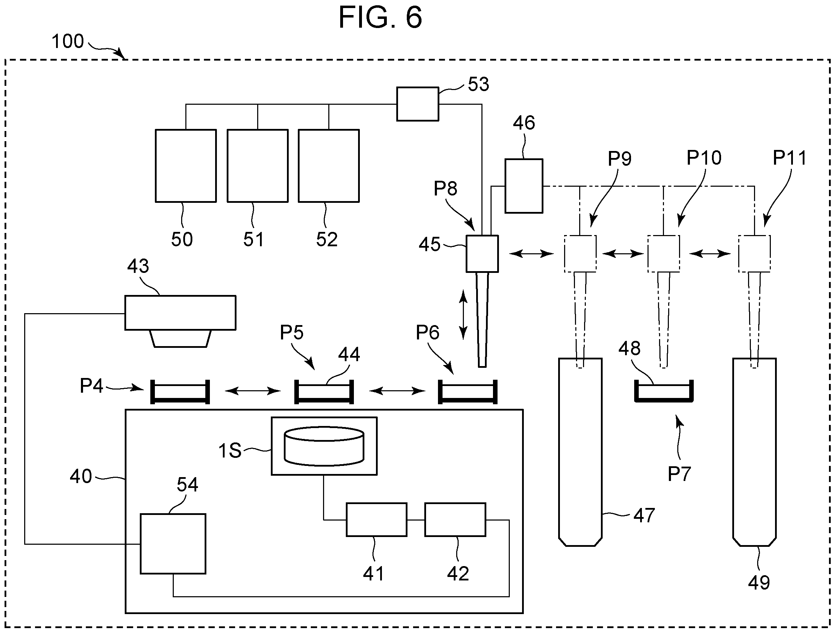

An example of the configuration of an ultrasonic detachment device 100 according to the embodiment of the present invention will be described with reference to conceptual diagram in Fig. 6. The constituent elements in Fig. 6 are 100: cell detachment device, 1S: ultrasonic application portion, 40: culture substrate movement control portion, 41: amplifier, 42: function generator, 43: cell information acquisition portion (cell information analysis portion may be included), 44: cell culture substrate, 45: pipetting portion, 46: pipetter movement control portion, 47: waste liquid recovery portion, 48: detached cell recovery portion (cell culture substrate), 49: detached cell recovery portion (tube), 50: washing medium, 51: culture medium, 52: cell detachment liquid, 53: liquid transport control portion, and 54: ultrasonic application determination portion (cell information analysis portion may be included). In this regard, positions are P4: position of culture substrate when cell information is acquired, P5: position of culture substrate when cell is detached (set in ultrasonic application portion), P6: position of culture substrate when cell detachment liquid is added, medium is exchanged, or cell is recovered, P7: position of new culture substrate when detached cell is seeded in new substrate, P8: position of pipetter when cell detachment liquid is added, medium is exchanged, or cell is recovered, P9: position of pipetter when medium or cell suspension liquid is discarded, P10: position of pipetter when cell suspension liquid is seeded in dish, and P11: position of pipetter when cell suspension liquid is recovered in tube.

The culture substrate movement control portion 40 is a unit to hold and move the culture substrate 44.

The culture substrate movement control portion 40 may include a warmer to control the temperature and the humidity of the cell detachment environment, and examples include a warming unit capable of maintaining 37°C and a humidification unit. To move the culture substrate 44, for example, an XYZ automated stage is used. Alternatively, a robot arm to hold and move the culture substrate 44 may be used. The culture substrate movement control portion 40 horizontally moves the culture substrate 44 between the position P4, the position P5, and the position P6. At the position P4, the information, such as a phase contrast image, of the cell cultured in the culture substrate 44 can be acquired by the cell information acquisition portion 43. At the position P5, an ultrasonic wave is applied to the culture substrate 44 by the ultrasonic application portion 1S. In addition, at the position P6, a solution in the culture substrate 44 is recovered or various solutions (also referred to as media) are added to the culture substrate 44 by the pipetting portion 45. The pipetting portion 45 may include a mechanism that manually or automatically discharges or suctions the medium. There is no limitation regarding the configuration of the pipetting portion 45 provided that a liquid can be transported, and, for example, a tube pump may be used. In this regard, a plurality of pipetting portions may be disposed and may be independently disposed in accordance with the type of the medium and the role.

Explanations will be further provided with reference to an example of a cell detachment process by using an ultrasonic wave.

The cell is cultured by the culture substrate 44, and the culture substrate 44 is arranged at the position P6 at a favorable timing of cell detachment. At an appropriate time, the culture substrate 44 is arranged at the position P4 by using the culture substrate movement control portion 40, and the manner of the cell may be observed by the cell information acquisition portion 43. Regarding the culture substrate 44 arranged at the position P6, the cell is adherent to the substrate. At first, the medium must be removed to be exchanged for the cell detachment liquid. Therefore, at the position P6, the medium in the culture substrate 44 is recovered by the pipetting portion 45. The recovered medium is discarded to the waste liquid recovery portion 47 at the position P9. Various media are stocked in the washing medium 50, the culture medium 51, and the cell detachment liquid 52 and are transported to the pipetting portion 45 by the liquid transport control portion 53. The pipetting portion moves in the vertical direction and, therefore, can access the culture substrate 44. A lid opening and closing system of the culture substrate 44 may be appropriately included. After the medium is removed, the cell adherent to the substrate may be appropriately washed. At the position P6, into the culture substrate 44 by the pipetting portion 45, the washing medium 50 is transported to the pipetting portion 45 by the liquid transport control portion 53, and at the position P6, the washing medium 50 is added to the culture substrate 44 by the pipetting portion 45. After the washing medium 50 is removed in the same manner akin to that of the above-described medium removal method, at the position P6, the cell detachment liquid 52 is added to the culture substrate 44 by the pipetting portion 45.

Subsequently, the culture substrate movement control portion 40 moves the culture substrate 44 to the position P4. Herein, the cell is incubated in the cell detachment liquid while the cell information is acquired by the cell information acquisition portion 43. At an appropriate timing or during the incubation, the cell information is acquired by the cell information acquisition portion 43, the obtained cell information is analyzed, and whether movement to the next detachment step is performed is determined. The determination is performed in the ultrasonic application determination portion 54. For example, when the cell information does not exceed a threshold value set in advance, incubation is continued. When the cell information is more than the threshold value, the ultrasonic application determination portion 54 determines to perform ultrasonic application, instructs the culture substrate movement control portion 40 to move the culture substrate 44, and sends an instruction to activate the ultrasonic application portion 1S, the function generator 42, and the amplifier 41.

Next, the culture substrate movement control portion 40 moves the culture substrate 44 to the position P5. Herein, the culture substrate 44 is set into the ultrasonic application portion 1S. A form in which an ultrasonic detachment unit is combined with the function generator 42 and the amplifier 41 that input an optional frequency, voltage, and the like to the ultrasonic detachment unit is exemplified. An ultrasonic wave is applied from the ultrasonic application portion 1S to the culture substrate 44, and the cell adherent to the culture substrate 44 is detached. The culture substrate 44 may be appropriately moved to the position P4 to monitor a detachment state of the cell by using the cell information acquisition portion 43.

After detachment of the cell is completed, the culture substrate movement control portion 40 moves the culture substrate 44 to the position P6. At the position P6, the cell detachment liquid containing the detached cell in the culture substrate 44 is recovered by the pipetting portion 45. At the position P10, the cell detachment liquid containing the detached cell is seeded in the detached cell recovery portion 48, for example, a new cell culture substrate disposed at the position P7. Alternatively, the cell detachment liquid is recovered in the tube 49 serving as the detached cell recovery portion at the position P11. These recovered cells are used for an assay of cell culture, cell measurement, cell marking, or the like thereafter.

The cell detachment device by using an ultrasonic wave according to the embodiment of the present invention may further include a control portion to control the above-described cell detachment process. The control portion is a means to control the action of each portion in the cell detachment device 100. For example, the control portion may be formed from a computer including a computation treatment portion such as CPU, memory such as RAM, and a storage portion such as a hard disk drive. The control portion may include an operation portion. The operation portion may include a display portion, an input portion, and the like. The display portion can display image data output from the control portion and various information relating to the action of the cell detachment device 100. For example, a liquid crystal display is used for the display portion. The input portion accepts various instruction inputs from a user. For example, a keyboard or a mouse is used for the input portion. Both function of the display portion and the function of the input portion may be a single unit such as a touch panel display.

The embodiment according to the present invention may be realized by executing one or more functions of the above-described embodiment by a computer of a system or a device (for example, application specific integrated circuit (ASIC)) to read and execute a computer-executable instruction (for example, at least one program) stored in a storage medium and/or by a method executed by a computer of a system or a device including one or more circuit to execute one or more functions of the above-described embodiment, by reading and executing the computer-executable instruction from the storage medium to, for example, execute one or more functions of the above-described embodiment, and/or by a computer of a system or a device while controlling one or more circuits to execute one or more functions of the above-described embodiment. The computer can include at least one processor (for example, central processing unit (CPU) and microprocessor unit (MPU)) and can include a network of independent computers or individual processors to read and execute computer-executable instructions. The computer-executable instruction may be provided to the computer from, for example, the network or the storage medium. The storage medium may include at least one of, for example, hard disks, random access memory (RAM), read only memory (ROM), storage devices of distributed computing systems, optical disks (compact disc (CD), digital versatile disc (DVD), Blu-ray Disc (BD)), flash memory devices, and memory cards.

(Examples)

The present invention will be described below in more detail with reference to Examples and Comparative examples, but the present invention is not limited to these.

[Example 1]

(Culture of cell on substrate)

An adenocarcinomic human alveolar basal epithelial A549 cell (hereafter simply referred to as A549 cell) was seeded at a density of 10,000 cells/cm$^2$ in a Φ60 polystyrene dish (produced by Corning) and cultured in an environment of 37°C and a $CO_2$ concentration of 5%. A medium in which 10% of Fetal Bovine Serum (produced by Sigma-Aldrich) and 1% of penicillin-streptomycin (10,000 U/ml, produced by Thermo Fisher Scientific) were added to DMEM (produced by Thermo Fisher Scientific) was used. Culture was performed for 48 hours, and the manner of the cell was observed by using a phase contrast microscope to check cell adhesion and growth. A cell occupation area in the dish was about 80%.

(Detachment of cell)

The medium in the dish was removed, and phosphate buffered saline (PBS(-), produced by Thermo Fisher Scientific) serving as a detachment liquid was added to the dish. Subsequently, the dish was set on the stage of a phase contrast microscope, and a phase contrast image was acquired. The cell areas of a plurality of cells were calculated from the phase contrast image. Image processing software ImageJ (National Institutes of Health, NIH) was used for measurement of the cell area to perform quantification. The quantified cell areas were averaged. The area of the cell immediately after coming into contact with the cell detachment liquid (incubation time of zero) was denoted by A1.
Thereafter, the dish was placed in an incubator at 37°C, and incubation was performed. The incubation time is expressed as an incubation time (min). After a lapse of a predetermined incubation time (min), the dish was removed from the incubator and set on the stage of a phase contrast microscope. The phase contrast image was acquired, and the cell area was quantified in the manner akin to that described above. The area of the cell with an incubation time t in the cell detachment liquid is denoted by A2. A cell area change rate X was determined by Formula (II) below.

$$X = (A1 - A2)/A1 \quad (II)$$

After the cell area change rate X was determined, the dish was set in the ultrasonic detachment device, and the cell was detached by performing sweep vibration (frequency of 22 to 27 kHz, sweep period of 1 s, and voltage of 200 V) at an environmental temperature of 37°C for 3 min.
The detached cell was recovered. Thereafter, the number of cells was measured using a hemocytometer, and a cell survival rate was calculated using a vitality assay by trypan blue staining. In addition, regarding the dish after ultrasonic detachment, all cells not detached by the ultrasonic wave were detached using a cell scraper, and the number of cells was measured using a hemocytometer. The sum of the number of cells detached by the ultrasonic wave and the number of cells detached thereafter by the cell scraper was denoted by a total number of detached cells, and a ratio of the number of cells detached by the ultrasonic wave to the total number of detached cells was denoted by detachment efficiency and calculated.
Table 1 presents the incubation time (min), the cell area change rate X, the detached cell survival rate (%), and the cell detachment efficiency (%).

[Table 1]

Table 1

| Incubation time (min) | Cell area change rate X | Detached cell survival rate (%) | Cell detachment efficiency (%) |
|---|---|---|---|
| none | 0 | 53.0 | 1 |
| 0.5 | 0.11 | 80.2 | 40.3 |
| 1 | 0.15 | 84.3 | 74.4 |

(continued)

| Incubation time (min) | Cell area change rate X | Detached cell survival rate (%) | Cell detachment efficiency (%) |
|---|---|---|---|
| 3 | 0.45 | 96.8 | 96.8 |
| 5 | 0.52 | 97.0 | 95.1 |
| 10 | 0.62 | 96.0 | 96.3 |
| 20 | 0.70 | 92.2 | 97.5 |

As is clear from Table 1, the cell area change rate increased over time with weakening adhesion between the cell and the substrate. For example, the cell area change rate was 0.15 when incubation was 1 min, was 0.52 when incubation was 5 min, and further increased to 0.7 when incubation time was 20 min.

The detachment liquid was added to the cell without performing incubation, and ultrasonic detachment was performed immediately. As a result, almost no cells were detached. In this regard, the survival rate of a small number of detached cells was low. When the incubation time was increased, the cell detachment efficiency was improved. The same applied to the cell survival rate.

When the incubation time was increased to 20 min, the cell survival rate tended to decrease.

It was found from the above-described results that when the cell is subjected to ultrasonic detachment, the time of incubating the cell with the detachment liquid has significant influence on the cell survival rate and the cell detachment efficiency. Herein, in a more practical sense, the cell area change rate to ensure compatibility between a high cell survival rate and a high detachment efficiency is 0.45 or more.

That is, setting the threshold value of the cell area change rate X to be, for example, 0.4 in advance and monitoring X over time enable the ultrasonic application timing for ultrasonic detachment to be determined. In this regard, the threshold value of the cell area change rate X may be set to be 0.45 or more. In the present Example, when the threshold value of X was set to be 0.4 in the above-described example, in Step [3] during cell detachment, the cell information is measured, and movement to Step [4] can be determined at a point in time when X reaches 0.4. Since X may be changed in accordance with the cell species and the culture condition, the threshold value may be set in advance by acquiring experimental data.

[Example 2]

The relationship between the incubation time (min), the brightness value of the phase contrast image, the detached cell survival rate (%), and the cell detachment efficiency (%) was examined in the manner akin to that of Example 1 except that the cell information was set to be the brightness value of the phase contrast image. Regarding calculation of the brightness value of the phase contrast image, the value can be determined by binarizing the phase contrast image and measuring an average brightness value by using image processing software.

The results are presented in Table 2.

[Table 2]

Table 2

| Incubation time (min) | Brightness value of phase contrast image | Detached cell survival rate (%) | Cell detachment efficiency (%) |
|---|---|---|---|
| none | 0 | 53 | 1 |
| 0.5 | 7.2 | 80.2 | 40.3 |
| 1 | 32.2 | 84.3 | 74.4 |
| 5 | 127.2 | 97 | 95.1 |
| 10 | 127.2 | 96 | 96.3 |

As is clear from Table 2, the brightness value of the phase contrast image increased over time with weakening adhesion between the cell and the substrate. The reason for this is that a halo region having a high image brightness value increases in accordance with a change in the shape of the cell in an adherent state due to weakening of cell adhesion.

The brightness value of the phase contrast image was 7.2 when incubation was 0.5 min, was 32.2 when incubation was 1 min, and further increased to 127.2 when incubation time was 5 min.

As in Example 1, it was found that the time of incubating the cell with the detachment liquid has significant influence on the cell survival rate and the cell detachment efficiency. Herein, in a more practical sense, the brightness value of the phase contrast image to ensure compatibility between a high cell survival rate and a high detachment efficiency is about 127.

That is, setting the threshold value of the brightness value of the phase contrast image to be 127 in advance and monitoring the brightness value over time enable the ultrasonic application timing for ultrasonic detachment to be determined.

Regarding the value of the threshold value in the present Example, various parameters and threshold values thereof are selected and set in accordance with the type of the cell, the environment of cell culture, the type of the cell information, the method for analyzing the cell information, the equipment to acquire the cell information, and the like.

[Example 3]

The relationship between the incubation time (min), the brightness value of the phase contrast image, the detached cell survival rate (%), and the cell detachment efficiency (%) was examined in the manner akin to that of Example 1 except that the cell information was set to be the average brightness value of the dish culture surface observed by oblique-incidence illumination. Regarding calculation of the average brightness value of the culture surface, the value can be determined by using image processing software.

The results are presented in Table 3.

[Table 3]

Table 3

| Incubation time (min) | Brightness value of dish culture surface | Detached cell survival rate (%) | Cell detachment efficiency (%) |
|---|---|---|---|
| none | 80.1 | 53 | 1 |
| 0.5 | 96.5 | 80.2 | 40.3 |
| 1 | 103.5 | 84.3 | 74.4 |
| 5 | 108.0 | 97 | 95.1 |
| 10 | 108.1 | 96 | 96.3 |

As is clear from Table 3, the brightness value of the dish culture surface observed by oblique-incidence illumination increased over time with weakening adhesion between the cell and the substrate. The reason for this is that scattered light at the interface between the substrate and the cell increases in accordance with a change in the shape of the cell in an adherent state due to weakening of cell adhesion.

The brightness value of the dish culture surface was 96.5 when incubation was 0.5 min, was 103.5 when incubation was 1 min, and further increased to 108.0 when incubation time was 5 min.

As in Example 1, it was found that the time of incubating the cell with the detachment liquid has significant influence on the cell survival rate and the cell detachment efficiency. Herein, in a more practical sense, the brightness value of the observation image of the dish culture surface by oblique-incidence illumination to ensure compatibility between a high cell survival rate and a high detachment efficiency is about 108.

That is, setting the threshold value of the brightness value to be 108 in advance and monitoring the brightness value over time enable the ultrasonic application timing for ultrasonic detachment to be determined.

Regarding the value of the threshold value of the observation image of the substrate culture surface by oblique-incidence illumination in the present Example, various parameters and threshold values thereof are selected and set. Various parameters and threshold values thereof are set in accordance with the type of the cell, the environment of cell culture, the type of the cell information, the type of the culture substrate, the illumination method such as the incident angle, the arrangement, and the intensity of the oblique-incidence illumination, the method for analyzing the cell information, the equipment to acquire the cell information, and the like.

[Example 4]

The relationship between the incubation time (min), the halo region area rate, the detached cell survival rate (%), and the cell detachment efficiency (%) was examined in the manner akin to that of Example 1 except that the cell information was set to be the halo region area rate. Regarding calculation of the halo region area rate, the value can be determined by binarizing the phase contrast image and measuring halo region area rate by using image processing software.
The results are presented in Table 4.

[Table 4]

Table 4

| Incubation time (min) | Halo region area rate from phase contrast image (%) | Detached cell survival rate (%) | Cell detachment efficiency (%) |
|---|---|---|---|
| 0.5 | 2.8 | 80.2 | 40.3 |
| 1 | 12.6 | 84.3 | 74.4 |
| 5 | 49.9 | 97.0 | 95.1 |

As is clear from Table 4, the halo region area rate from the phase contrast image increased over time with weakening adhesion between the cell and the substrate. The reason for this is that a halo region having a high image brightness value increases in accordance with a change in the shape of the cell in an adherent state due to weakening of cell adhesion.
The halo region area rate was 2.8% when incubation was 0.5 min, was 12.6% when incubation was 1 min, and further increased to 49.9% when incubation time was 5 min.
As in Example 2, it was found that the time of incubating the cell with the detachment liquid has significant influence on the cell survival rate and the cell detachment efficiency. Herein, in a more practical sense, the halo region area rate to ensure compatibility between a high cell survival rate and a high detachment efficiency is about 49.9%.
That is, setting the threshold value of the halo region area rate to be 49.9% in advance and monitoring the halo region area rate over time enable the ultrasonic application timing for ultrasonic detachment to be determined.
Regarding the value of the threshold value in the present Example, various parameters and threshold values thereof are selected and set in accordance with the type of the cell, the environment of cell culture, the type of the cell information, the method for analyzing the cell information, the equipment to acquire the cell information, and the like.

[Example 5]

(Culture of cell on substrate)

A mouse skeletal muscle myoblast cell (hereafter simply referred to as C2C12 cell) was seeded at a density of 10,000 cells/cm$^2$ in a $\Phi$35 polystyrene dish (produced by Corning) and cultured in an environment of 37°C and a $CO_2$ concentration of 5%. A medium in which 10% of Fetal Bovine Serum (produced by Sigma-Aldrich) and 1% of penicillin-streptomycin (10,000 U/ml, produced by Thermo Fisher Scientific) were added to DMEM (produced by Thermo Fisher Scientific) was used. Culture was performed for 48 hours, and the manner of the cell was observed by using a phase contrast microscope to check cell adhesion and growth. A cell occupation area in the dish was about 80%.

(Detachment of cell)

The medium in the dish was removed, and phosphate buffered saline serving as a detachment liquid was added to the dish. Thereafter, the dish was placed in an incubator at 37°C, and incubation was performed. After a lapse of a predetermined incubation time (min), the dish was removed from the incubator and set on the stage of a phase contrast microscope, and the phase contrast image was acquired. An area rate (%) of a region outside the cell in the phase contrast image was determined by quantifying the area of a region to which no cell adhered (area of a region outside the cell) by image analysis. Regarding calculation of the area rate, ImageJ is used, the region is set by setting the threshold value after the phase contrast image is binarized, and the region outside the cell can be quantified.
After the area rate of the region outside the cell was determined as the cell information, the dish was set in the ultrasonic detachment device, and the cell was detached by performing sweep vibration (frequency of 22 to 27 kHz, sweep period of 1 s, and voltage of 100 V) at an environmental temperature of 37°C for 3 min.

Regarding the detached cell, the cell survival rate was calculated in the manner akin to that of Example 1. In this regard, the cell detachment efficiency was evaluated by visually observing at least four positions by using a phase contrast microscope. In the field of view, a state in which an area occupied by the cell adherent to the dish is smaller than 20% is denoted by "high" detachment efficiency, a state in which the occupied area is 20% or more and less than 60% is denoted by "middle" detachment efficiency, and a state in which the occupied area is 60% or more is denoted by "low" detachment efficiency.

Table 5 presents the incubation time (min), the area rate of the region outside the cell (%), the detached cell survival rate (%), and the cell detachment efficiency.

[Table 5]

Table 5

| Incubation time (min) | Area rate of region outside cell (%) | Detached cell survival rate (%) | Cell detachment efficiency |
|---|---|---|---|
| 1 | 51.4 | 83.9 | low |
| 5 | 71.7 | 93.3 | middle |
| 10 | 79.0 | 93.6 | high |

As is clear from Table 5, the area rate of the region outside the cell from the phase contrast image increased over time with weakening adhesion between the cell and the substrate. The reason for this is that a region outside the cell increases in accordance with a change in the shape of the cell in an adherent state due to weakening of cell adhesion. As in Example 1, it was found that the time of incubating the cell with the detachment liquid has significant influence on the cell survival rate and the cell detachment efficiency. Herein, in a more practical sense, the area rate of the region outside the cell to ensure compatibility between a high cell survival rate and a high detachment efficiency is about 79%. That is, setting the threshold value of the area rate of the region outside the cell to be 79% in advance and monitoring the area rate of the region outside the cell over time enable the ultrasonic application timing for ultrasonic detachment to be determined. Regarding the value of the threshold value in the present Example, various parameters and threshold values thereof are selected and set in accordance with the type of the cell, the environment of cell culture, the type of the cell information, the method for analyzing the cell information, the equipment to acquire the cell information, and the like.

[Example 6]

Cell detachment was performed in the manner akin to that of Example 5 except that the detachment liquid was set to be a detachment liquid containing EDTA (0.1 mM) serving as a metal ion chelating agent and polyethylene glycol (average molecular weight of 4,000 and concentration of 1.0%). In addition, the relationship between the incubation time (min), the area rate of the region outside the cell, the detached cell survival rate (%), and the cell detachment efficiency was examined.

The results are presented in Table 6.

[Table 6]

Table 6

| Incubation time (min) | Area rate of region outside cell (%) | Detached cell survival rate (%) | Cell detachment efficiency |
|---|---|---|---|
| 1 | 69.5 | 79.3 | middle |
| 3 | 76.4 | 88.9 | high |
| 6 | 81.3 | 90.1 | high |

As is clear from Table 6, the area rate of the region outside the cell to ensure compatibility between a high cell survival rate and a high detachment efficiency is about 76%, as in Example 5. That is, setting the threshold value of the area rate of the region outside the cell to be 76% in advance and monitoring the area rate of the region outside the cell over time enable the ultrasonic application timing for ultrasonic detachment to be determined.

The disclosure of the present specification includes the following methods and configurations.

(Method 1)

A cell detachment method for detaching a cell from a substrate by applying vibration to the cell adherent to the substrate, the method including,

an incubation step of incubating the cell after bringing a cell detachment liquid containing substantially no proteolytic enzyme into contact with the cell,
an information acquisition step of acquiring information relating to the incubated cell,
a determination step of determining a time when vibration is applied to the incubated cell in accordance with the information relating to the cell, and
a detachment step of applying vibration to the incubated cell.

(Method 2)

The cell detachment method according to Method 1, wherein the vibration is vibration in an ultrasonic band.

(Method 3)

The cell detachment method according to Method 1 or Method 2, wherein the cell detachment liquid contains a metal ion chelating agent.

(Method 4)

The cell detachment method according to any one of Methods 1 to 3, wherein the cell detachment liquid contains polyethylene glycol.

(Method 5)

The cell detachment method according to any one of Methods 1 to 4, wherein the incubation step is performed in an environment of 37°C.

(Method 6)

The cell detachment method according to any one of Methods 1 to 5,

wherein the information acquisition step includes a step of acquiring an image of the cell, and
the information relating to the cell is at least one of an area of a region of the cell, an area of a halo region of the cell, and a brightness value of the region of the cell in the image of the cell.

(Method 7)

The cell detachment method according to Method 6, wherein the brightness value is a brightness value of a cell culture surface of the substrate observed by oblique-incidence illumination.

(Configuration 1)

A program causing a computer to execute the cell detachment method according to any one of Methods 1 to 7.

(Configuration 2)

A cell detachment system for detaching a cell from a substrate by applying vibration to the cell adherent to the substrate, the system including,

an incubation portion to incubate the cell after bringing a cell detachment liquid containing substantially no proteolytic enzyme into contact with the cell,
an information acquisition portion to acquire information relating to the incubated cell,
a determination portion to determine a time when vibration is applied to the incubated cell in accordance with the information relating to the cell, and
a detachment portion to apply vibration to the incubated cell.

(Configuration 3)

An information processing device for controlling a cell detachment device to detach a cell from a substrate by applying vibration to the cell adherent to the substrate, the information processing device including,

an information acquisition portion to acquire information relating to the cell in contact with a cell detachment liquid containing substantially no proteolytic enzyme, and
a determination portion to determine a time when vibration is applied to the cell in accordance with the information relating to the cell.

The present invention is not limited to the above-described embodiments and can be variously changed and modified without departing from the spirit and scope of the present invention. Therefore, to apprise the public of the scope of the present invention, the following claims are appended.
This application claims the benefit of Japanese Patent Application No. 2022-013181 filed January 31, 2022 and No. 2022-190039 filed November 29, 2022, which are hereby incorporated by reference herein in their entirety.

**Claims**

1.  A cell detachment method for detaching a cell from a substrate by applying vibration to the cell adherent to the substrate, the method comprising:

    an incubation step of incubating the cell after bringing a cell detachment liquid containing substantially no proteolytic enzyme into contact with the cell;
    an information acquisition step of acquiring information relating to the incubated cell;
    a determination step of determining a time when vibration is applied to the incubated cell in accordance with the information relating to the cell; and
    a detachment step of applying vibration to the incubated cell.

2.  The cell detachment method according to Claim 1, wherein the vibration is vibration in an ultrasonic band.

3.  The cell detachment method according to Claim 1, wherein the cell detachment liquid contains a metal ion chelating agent.

4.  The cell detachment method according to Claim 1, wherein the cell detachment liquid contains polyethylene glycol.

5.  The cell detachment method according to Claim 1, wherein the incubation step is performed in an environment of 37°C.

6.  The cell detachment method according to Claim 1,

    wherein the information acquisition step includes a step of acquiring an image of the cell, and
    the information relating to the cell is at least one of an area of a region of the cell, an area of a halo region of the cell, and a brightness value of the region of the cell in the image of the cell.

7.  The cell detachment method according to Claim 6, wherein the brightness value is a brightness value of a cell culture surface of the substrate observed by oblique-incidence illumination.

8.  A program causing a computer to execute the cell detachment method according to any one of Claims 1 to 7.

9.  A cell detachment system for detaching a cell from a substrate by applying vibration to the cell adherent to the substrate, the system comprising:

an incubation portion to incubate the cell after bringing a cell detachment liquid containing substantially no proteolytic enzyme into contact with the cell;
an information acquisition portion to acquire information relating to the incubated cell;
a determination portion to determine a time when vibration is applied to the incubated cell in accordance with the information relating to the cell; and
a detachment portion to apply vibration to the incubated cell.

10. An information processing device for controlling a cell detachment device to detach a cell from a substrate by applying vibration to the cell adherent to the substrate, the information processing device comprising:

an information acquisition portion to acquire information relating to the cell in contact with a cell detachment liquid containing substantially no proteolytic enzyme; and
a determination portion to determine a time when vibration is applied to the cell in accordance with the information relating to the cell.

# FIG. 1

```
        ┌─────────────┐
        │    START    │
        └─────────────┘
               │
               ▼
┌──────────────────────────────┐
│      INCUBATING CELL         │────S101
└──────────────────────────────┘
               │
               ▼
┌──────────────────────────────┐
│    ACQUIRING INFORMATION     │────S102
│      RELATING TO CELL        │
└──────────────────────────────┘
               │
               ▼
┌──────────────────────────────┐
│    DETERMINING TIME WHEN     │────S103
│   ULTRASONIC WAVE IS APPLIED │
└──────────────────────────────┘
               │
               ▼
┌──────────────────────────────┐
│ APPLYING ULTRASONIC WAVE TO CELL │────S104
└──────────────────────────────┘
               │
               ▼
        ┌─────────────┐
        │     END     │
        └─────────────┘
```

# FIG. 2

**START**

ADDING DETACHMENT LIQUID
TO CULTURE BASE MATERIAL — S201

ACQUIRING FIRST IMAGE BY
PICKING UP IMAGE AT PREDETERMINED
POSITION IN CULTURE BASE MATERIAL — S202

DETERMINING CELL AREA A1 — S203

INCUBATING FOR PREDETERMINED TIME — S204

ACQUIRING SECOND IMAGE BY
PICKING UP IMAGE AT THE SAME
POSITION AS POSITION OF FIRST IMAGE — S205

DETERMINING CELL AREA A2 — S206

DETERMINING CHANGE RATE
X (A1 − A2/A1) OF CELL AREA — S207

S208
CHANGE RATE X >
THRESHOLD VALUE?
NO
YES

APPLYING VIBRATION DUE
TO ULTRASONIC WAVE — S209

**END**

# FIG. 3

```
            ┌─────────────┐
            │    START    │
            └──────┬──────┘
                   │
   ┌──────────────▼──────────────┐
   │  ADDING DETACHMENT LIQUID   │──S301
   │   TO CULTURE BASE MATERIAL  │
   └──────────────┬──────────────┘
                  │
   ┌─────────────►│
   │  ┌───────────▼──────────────┐
   │  │      INCUBATING FOR       │──S302
   │  │    PREDETERMINED TIME     │
   │  └───────────┬──────────────┘
   │              │
   │  ┌───────────▼──────────────┐
   │  │ PICKING UP PHASE CONTRAST │──S303
   │  │          IMAGE            │
   │  └───────────┬──────────────┘
   │              │
   │  ┌───────────▼──────────────┐
   │  │ CALCULATING BRIGHTNESS    │──S304
   │  │ VALUE OR HALO REGION      │
   │  │ AREA RATE X OF CELL       │
   │  └───────────┬──────────────┘
   │              │
   │        ┌─────▼─────┐  S305
   │   NO   │ BRIGHTNESS│
   └────────┤ VALUE OR  │
            │ HALO REGION
            │ AREA RATE X >
            │ THRESHOLD VALUE?
            └─────┬─────┘
                  │ YES
   ┌──────────────▼──────────────┐
   │   APPLYING VIBRATION DUE     │──S306
   │     TO ULTRASONIC WAVE       │
   └──────────────┬──────────────┘
                  │
            ┌─────▼─────┐
            │    END    │
            └───────────┘
```

# FIG. 4

```
            ┌─────────────┐
            │    START    │
            └──────┬──────┘
                   │
        ┌──────────▼──────────────┐
        │  ADDING DETACHMENT LIQUID│──── S601
        │  TO CULTURE BASE MATERIAL│
        └──────────┬──────────────┘
                   │
        ┌──────────▼──────────────┐
        │     INCUBATING FOR       │──── S602
        │   PREDETERMINED TIME     │
        └──────────┬──────────────┘
                   │
        ┌──────────▼──────────────┐
        │ PICKING UP OBLIQUE-INCIDENCE│── S603
        │ ILLUMINATION OBSERVATION IMAGE│
        └──────────┬──────────────┘
                   │
        ┌──────────▼──────────────┐
        │  CALCULATING BRIGHTNESS  │──── S604
        │  VALUE X OF CELL CULTURE │
        │  SURFACE OF BASE MATERIAL│
        └──────────┬──────────────┘
                   │
                   ▼           S605
    NO       ◇─────────────────◇
   ◄─────────  BRIGHTNESS VALUE X >
             THRESHOLD VALUE?
             ◇─────────────────◇
                   │ YES
        ┌──────────▼──────────────┐
        │   APPLYING VIBRATION DUE │──── S606
        │    TO ULTRASONIC WAVE    │
        └──────────┬──────────────┘
                   │
            ┌──────▼──────┐
            │     END     │
            └─────────────┘
```

# FIG. 5

110

1110

| 1101 | CPU |
| 1102 | RAM |
| 1103 | ROM |
| 1104 | HDD |

| COMMUNICATION I/F | 1105 |
| DISPLAY DEVICE | 1106 |
| INPUT DEVICE | 1107 |

# FIG. 6

EP 4 474 473 A1

# FIG. 7

```
┌─────────────────────────────────────┐
│   ┌───────────────────┐              │
│   │    INCUBATION      │ ～ 201       │
│   │     PORTION        │              │
│   └───────────────────┘              │
│                                      │
│   ┌───────────────────┐              │
│   │   INFORMATION      │              │
│   │   ACQUISITION      │ ～ 202    ～ 200
│   │     PORTION        │              │
│   └───────────────────┘              │
│                                      │
│   ┌───────────────────┐              │
│   │  DETERMINATION     │ ～ 203       │
│   │     PORTION        │              │
│   └───────────────────┘              │
│                                      │
│   ┌───────────────────┐              │
│   │   DETACHMENT       │ ～ 204       │
│   │     PORTION        │              │
│   └───────────────────┘              │
└─────────────────────────────────────┘
```

## INTERNATIONAL SEARCH REPORT

| International application No. |
|---|
| **PCT/JP2023/002363** |

### A. CLASSIFICATION OF SUBJECT MATTER

***C12N 5/07***(2010.01)i; ***C12N 5/071***(2010.01)i; ***C12M 1/00***(2006.01)i; ***C12M 1/42***(2006.01)i
FI:    C12N5/07; C12M1/42; C12M1/00 C; C12N5/071

According to International Patent Classification (IPC) or to both national classification and IPC

### B. FIELDS SEARCHED

Minimum documentation searched (classification system followed by classification symbols)

C12N5/00-5/28; C12M1/00-3/10

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN); PubMed

### C. DOCUMENTS CONSIDERED TO BE RELEVANT

| Category* | Citation of document, with indication, where appropriate, of the relevant passages | Relevant to claim No. |
|---|---|---|
| Y | KURASHINA, Y. et al., Enzyme-free release of adhered cells from standard culture dishes using intermittent ultrasonic traveling waves, COMMUNICATIONS BIOLOGY, 2019, vol. 2: 393, pp. 1-11<br>in particular, p. 1, line 1 to line 1 from the bottom, p. 9, left column, lines 8-39, fig. 1 | 1-10 |
| Y | JP 2020-028287 A (TOSOH CORP.) 27 February 2020 (2020-02-27)<br>claims, paragraphs [0036], [0047], examples | 1-10 |
| Y | JP 2018-201403 A (ZEON CORP.) 27 December 2018 (2018-12-27)<br>claims, paragraph [0041] | 1-10 |
| Y | JP 2014-513556 A (LONZA WALKERSVILLE INC.) 05 June 2014 (2014-06-05)<br>claims, examples | 1-10 |
| Y | JP 2020-048446 A (NATIONAL INSTITUTE FOR MATERIALS SCIENCE) 02 April 2020 (2020-04-02)<br>claims, examples, table 1, fig. 6 | 1-10 |
| Y | WO 2007/136073 A1 (NIKON CORP.) 29 November 2007 (2007-11-29)<br>claims, paragraphs [0008]-[0017], fig. 1-8 | 1-10 |

☑ Further documents are listed in the continuation of Box C.      ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **03 April 2023** | **11 April 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

International application No.

**PCT/JP2023/002363**

**C. DOCUMENTS CONSIDERED TO BE RELEVANT**

| Category* | Citation of document, with indication, where appropriate, of the relevant passages | Relevant to claim No. |
|---|---|---|
| A | WO 2016/047368 A1 (SCREEN HOLDINGS CO., LTD.) 31 March 2016 (2016-03-31)<br>    entire text | 1-10 |
| A | JP 2006-314204 A (CHIYODA CORP.) 24 November 2006 (2006-11-24)<br>    entire text | 1-10 |
| A | 竹村 研治郎, 超音波技術を基盤とした細胞培養システム, 日本機械学会誌, 2020, vol. 123, pp. 22-25 non-official translation (Cell cultivation system based on ultrasonic technology. Mechanical Engineering Journal.)<br>    entire text | 1-10 |

Form PCT/ISA/210 (second sheet) (January 2015)

**INTERNATIONAL SEARCH REPORT**
Information on patent family members

International application No.

**PCT/JP2023/002363**

| Patent document cited in search report | | | Publication date (day/month/year) | Patent family member(s) | Publication date (day/month/year) |
|---|---|---|---|---|---|
| JP | 2020-028287 | A | 27 February 2020 | (Family: none) | |
| JP | 2018-201403 | A | 27 December 2018 | (Family: none) | |
| JP | 2014-513556 | A | 05 June 2014 | WO 2012/158899 A1 claims, examples | |
| JP | 2020-048446 | A | 02 April 2020 | (Family: none) | |
| WO | 2007/136073 | A1 | 29 November 2007 | US 2009/0081769 A1 claims, paragraphs [0036]-[0045], fig. 1-8 | |
| WO | 2016/047368 | A1 | 31 March 2016 | (Family: none) | |
| JP | 2006-314204 | A | 24 November 2006 | (Family: none) | |

Form PCT/ISA/210 (patent family annex) (January 2015)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016047368 A
- JP 2003235540 A
- WO 2007136073 A
- JP 2008092857 A
- JP 2006314204 A
- JP 2022013181 A
- JP 2022190039 A